# EUROPEAN PATENT APPLICATION

(11) **EP 0 612 506 A2**
(43) Date of publication of application: **31.08.1994**
(21) Application number: 94300943.1
(22) Date of filing: 09.02.1994
(51) Int. Cl.: A61B 17/32

(54) **Surgical scalpel for making controlled depth and width inscisions**

(30) Priority: 26.02.1993 US 23521
(71) Applicant: Becton Dickinson and Company, Franklin Lakes, New Jersey 07417-1880 (US)
(72) Inventor: Newman, Craig D., New York, New York 10024 (US)
(74) Representative: Ruffles, Graham Keith

(57) **Abstract**

A surgical scalpel (60) capable of making a controlled depth and width incision for inserting a trocar during endoscopic surgery. The scalpel (60) includes a handle (61) and a shield (62) slidably mounted on the handle between an extended position and a retracted position. When the shield is in the retracted position, a predetermined portion of a blade (80) mounted on the handle extends beyond the distal end (73) of the shield. The portion of blade extending beyond the distal end (73) of the shield is correlated to the position of the shank (63) in the shield so that a controlled depth and width incision can be made using the portion of the blade extending beyond the distal end of the shield. The distal end of the shield, therefore, provides a means for stopping the further insertion of the blade into a cavity wall and causes a predetermined depth and width starter incision to be made.

## Description

### BACKGROUND OF THE INVENTION

### 1. Field of the Invention

The present invention relates to a laparoscopic starter scalpel and, more particularly, relates to a surgical scalpel that makes a controlled depth and width incision for the insertion of a trocar.

### 2. Background Description

As practitioners-in-the-art of endoscopic surgery are aware, it has long been a goal to have an efficient surgical tool for making a controlled depth and width incision in the wall of an anatomical cavity for inserting a trocar into the cavity.

Trocars are conventional operating instruments that are used to form a passageway through the wall of an anatomical cavity into the cavity. After a trocar has been inserted into the cavity other operating instruments such as endoscopes and arthoscopes can be inserted into the cavity through the passageway to perform medical procedures within the cavity. However, before a trocar can be inserted into a cavity a starter incision must be made in the wall of the cavity.

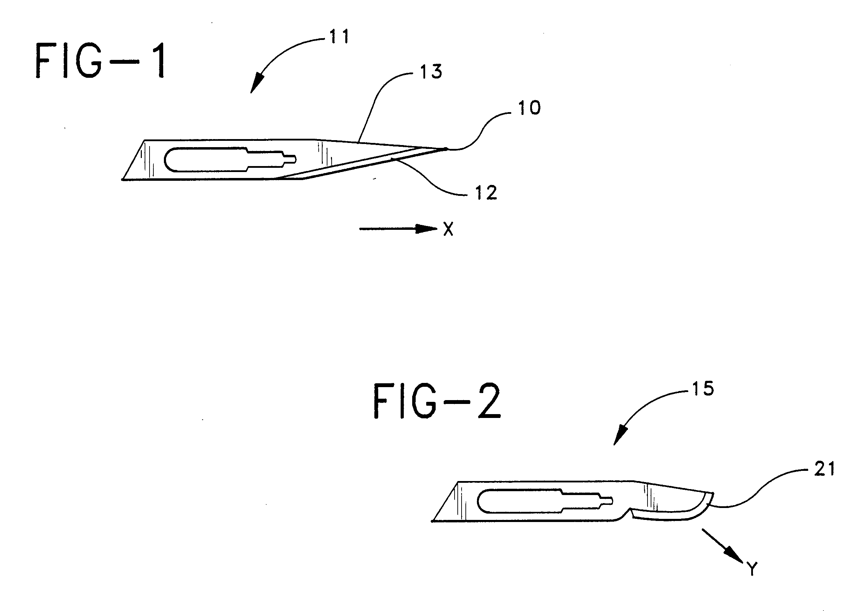

Operating room personnel commonly use a conventional surgical scalpel having a blade similar to blades 11 and 15 shown in Figs. 1 and 2, respectively, to make the starter incision. However, since blades 11 and 15 only have one cutting edge and require the user to visually determine whether the size of the incision is sufficient to receive a trocar, these blades do not provide the cutting capability and control that is necessary when making a starter incision for receiving a trocar.

For example, to make a starter incision in a wall of an anatomical cavity using a scalpel with blade 11, shown in Fig. 1, pressure is applied in the X direction with point 10 being in pressure contact with the wall of the cavity. As point 10 pierces the wall an incision is made in the wall by cutting edge 12. Opposite edge 13 of blade 11 does not have a cutting edge and therefore does not perform cutting. Pressure in the X direction is applied until the desired width of the incision is visually observed by the user.

Similarly, when making a starter incision in the wall of a cavity using a scalpel with blade 15, shown in Fig. 2, only cutting edge 21 performs cutting. Pressure is applied to blade 15 in the Y direction as cutting edge 21 of blade 15 is moved over the area of the cavity wall in which an incision is desired. The pressure on and movement of blade 15 continues until the desired size of the incision is visually observed by the user.

Other scalpels that could be used to make an incision for inserting a trocar are shown in U.S. Patent No. 3,906,626 (Riuli) and U.S. Patent No. 4,759,363 (Jensen). Riuli relates to a disposable surgical scalpel having a slidable sheath that temporarily covers the blade of the scalpel when the scalpel is not in use and permanently covers the blade when the scalpel is to be disposed of. Jensen relates to a scalpel having a removable guard that defines the depth of a cut when attached to the scalpel. However, the scalpels described in Riuli and Jensen only have one cutting edge and require the user to visually determine the final width of the incision.

Since blades 11 and 15 and the Riuli and Jensen scalpels require a user to visually determine the size of an incision, these devices do not provide the control that is necessary to make starter incisions having a specific depth and width for receiving a trocar. In addition, each of the blades discussed above have only one cutting edge. Therefore, there is the need for a surgical scalpel that efficiently makes a starter incision using two cutting edges and controls the depth and width of the incision so that a trocar can be properly inserted through the incision into an anatomical cavity.

### SUMMARY OF THE INVENTION

The present invention overcomes the problems identified in the background material by providing a surgical scalpel with twin cutting edges and means for controlling the depth and width of a starter incision needed for the insertion of a trocar.

A preferred embodiment of the surgical scalpel includes a handle with an arrow-shaped blade and a shield that extends to cover the blade and retracts to a predetermined position to expose a predetermined portion of the blade for making a predetermined depth and width starter incision. The shield functions as means for controlling both the width and depth of the incision and is adjustable to provide various predetermined positions corresponding to predetermined width and depth incisions for specific trocar sizes, e.g., 5mm, 10mm, 12mm. The arrow-shaped blade slides within the shield of the handle and includes twin cutting edges that permit the user to easily make the starter incision.

In addition, the shield is capable of extending over the blade to an extended position to cover the blade when passing the scalpel, and can be moved to a fully-extended and locked position so that the blade is permanently covered permitting the scalpel to be disposed of.

These and other aspects, features and advantages of the present invention will become apparent from the following detailed description taken in conjunction with the accompanying drawings.

### DESCRIPTION OF THE DRAWINGS

Fig. 1 is a side view of a conventional surgical blade;
Fig. 2 is a side view of another conventional surgical blade;
Fig. 3 is a side view of a preferred surgical scalpel of the present invention;
Fig. 4 is an enlarged side view of the preferred blade on the surgical scalpel shown in Fig. 3;
Fig. 5 is an enlarged side view of an alternate preferred arrow-shaped blade for use on the surgical scalpel shown in Fig. 3;
Fig. 6 is an isometric view of another preferred surgical scalpel of the present invention with a slidable shield in an extended position;
Fig. 7 is a cross-sectional view of the scalpel shown in Fig. 6;
Fig. 8 is an isometric view of the scalpel shown in Fig. 6 with the shield in a retracted position;
Fig. 9 is a cross-sectional view of the scalpel shown in Fig. 8;
Fig. 10 is an isometric view of the scalpel shown in Fig. 6 with the shield in a fully-extended and locked position;
Fig. 11 is a cross-sectional view of the scalpel shown in Fig. 10;
Fig. 12 is an exploded isometric view of the scalpel shown in Fig. 6;
Fig. 13 is an isometric view of an alternative preferred surgical scalpel of the present invention with a slidable blade in an extended position; and
Fig. 14 is an isometric view of the scalpel shown in Fig. 13 with the slidable blade in a retracted position.

### DETAILED DESCRIPTION

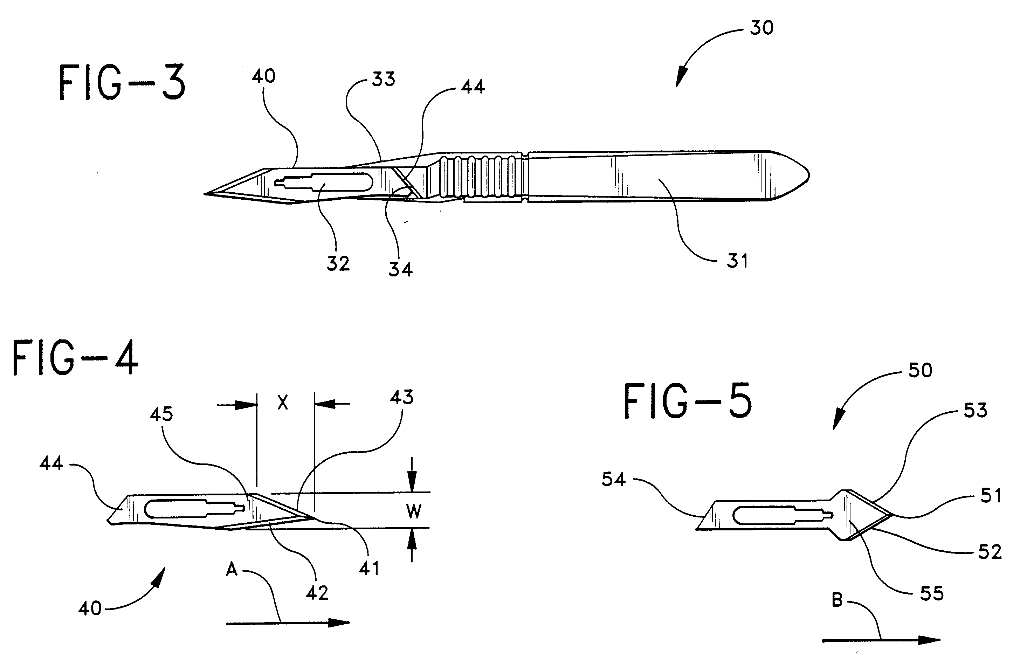

A preferred surgical scalpel 30 embodying the principles and concepts of the present invention is shown in Fig. 3 and includes a handle 31 having a boss 32 located at a distal end 33.

A blade 40 is attached to boss 32 on handle 31 and a foot 44 on blade 40 mates with a blade abutment 34 on handle 31 to secure blade 40 to handle 31. Blade 40 is attached to boss 32 by, e.g., mechanical, chemical or ultrasonic means, however, other means for attaching blade 40 to boss 32 could be used. As shown in Fig. 4, blade 40 includes a head portion 45 having a pair of cutting edges 42 and 43 that converge to a point 41. Cutting edges 42 and 43 permit a user to use scalpel 30 to efficiently make a controlled starter incision in a cavity wall of a patient by placing point 41 in pressure contact with the cavity wall and applying pressure in the A direction. As pressure is applied, cutting edges 42 and 43 cut the cavity wall on either side of point 41. To complete the starter incision, pressure is continuously applied in the A direction until the user observes that the width of the cut in the wall is the intended width for receiving a trocar of a predetermined size. For example, as shown in Fig. 4, head portion 45 of blade 40 preferably has a length, x, of 14.224mm and a width, w, of 7.762mm, however, other lengths and widths could also be used.

Fig. 5 shows an alternative preferred blade 50 having an arrow-shaped head 55. Arrow-shaped head 55 includes a pair of cutting edges 52 and 53 that converge at substantially the same angle to a point 51. Blade 50 is mountable on boss 32 of scalpel 30 in Fig. 3, in place of blade 40, with a foot 54 in contact with abutment 34 to provide an alternative preferred scalpel for making a starter incision for receiving a trocar.

When using scalpel 30 combined with blade 50 to make a starter incision, point 51 is placed in pressure contact with a patient's cavity wall with pressure applied in the B direction. As pressure is applied, cutting edges 52 and 53 begin to cut the cavity wall on both sides of point 51 and form an incision in the wall. Pressure is applied in the B direction until the user visually determines that the incision is the intended width for receiving a preselected trocar. The symmetrical arrow-shaped head 55 on blade 50 efficiently makes an incision that is symmetrical about point 51 and provides effective means for controlling the depth and width of the incision.

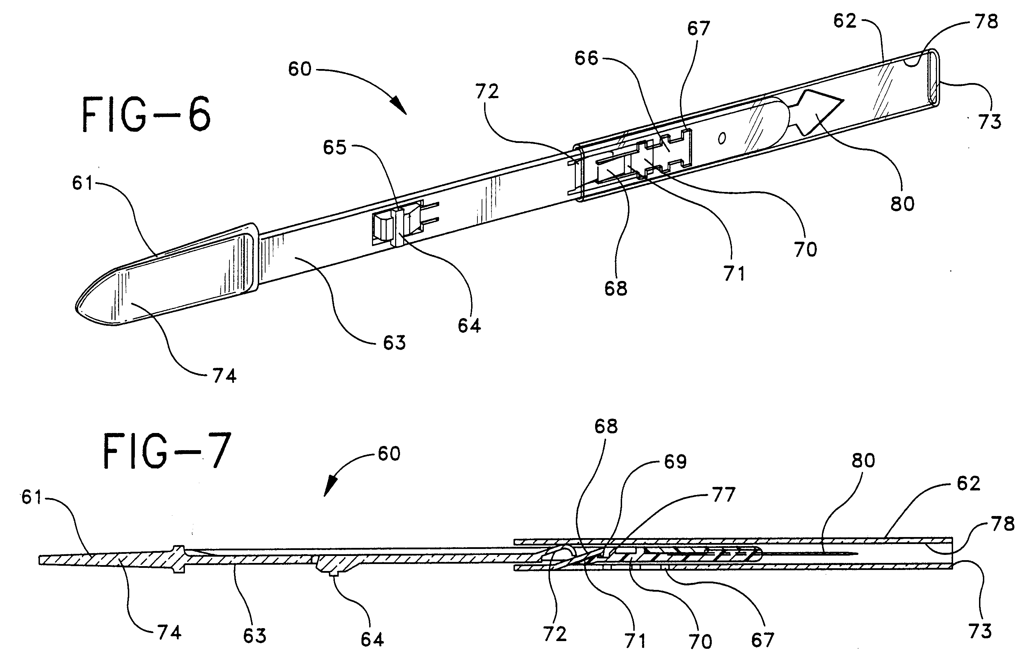

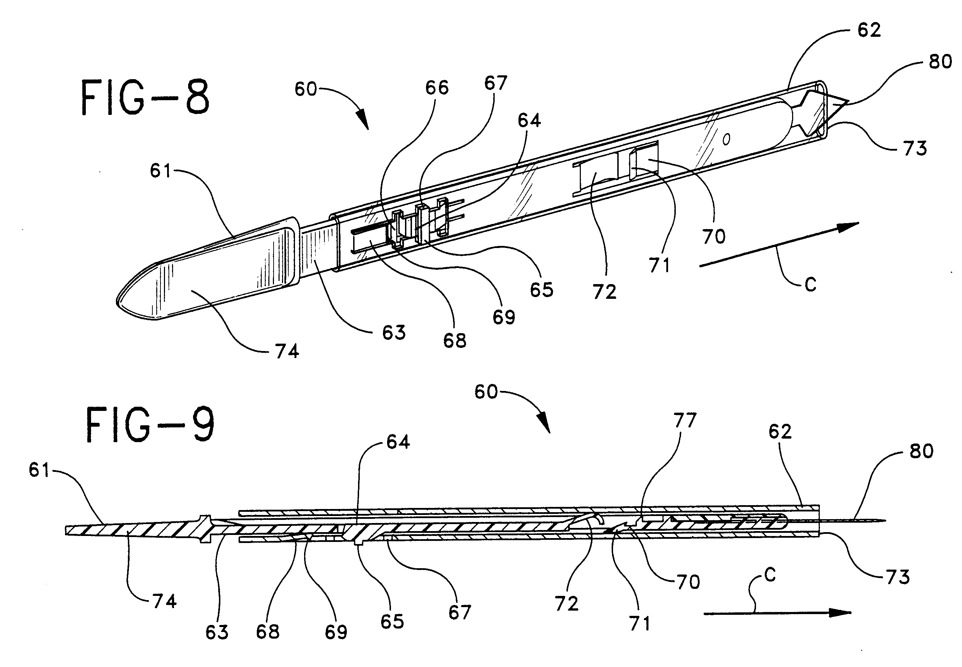

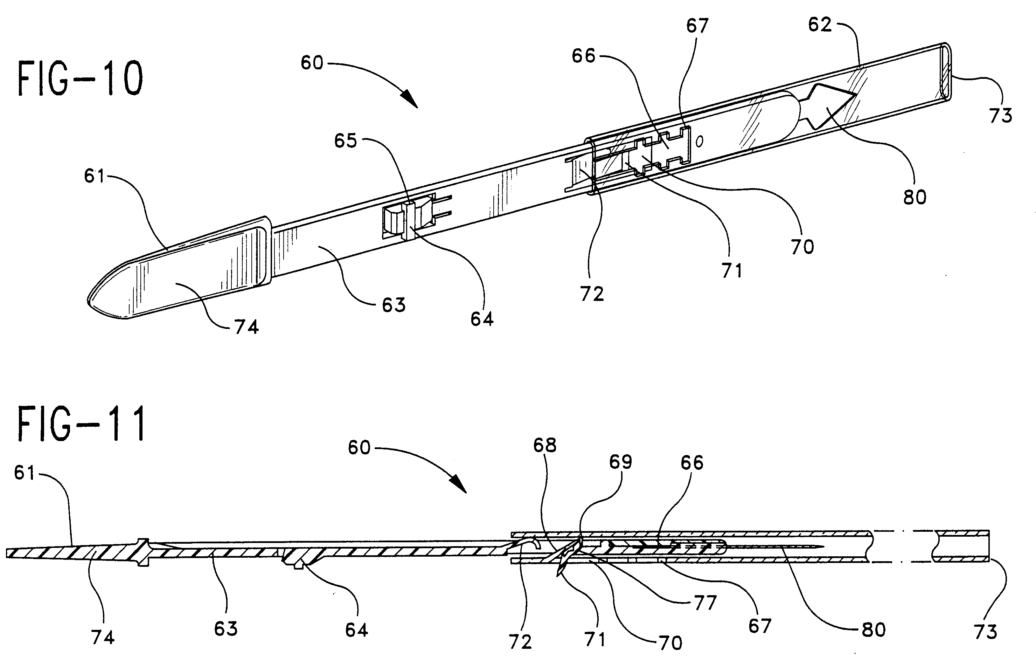

Another preferred embodiment of a starter scalpel according to the present invention is shown in Figs. 6-12. Surgical scalpel 60 in Fig. 6 includes a handle 61 and a shield 62 that is slidably mounted on handle 61 from an extended position shown in Fig. 6 to a retracted position shown in Fig. 8, and finally to a fully-extended and locked position showrrin Fig. 10 for disposal.

Handle 61 in Fig. 6 includes a butt 74 having a shank 63 extending longitudinally therefrom with a thickness that is smaller than butt 74 such that shield 62 can slide thereon. Shank 63 includes a resilient button 64 having a pair of shoulders 65 extending therefrom, a blade 80, a flexible arm 70 having a raised latch 71, and a pressure surface 72 that slides and makes contact with an internal surface 78 of shield 62.

Shield 62 includes a longitudinal opening 66 having three pairs of indents 67 along opposite longitudinal sides of opening 66, and a flexible tab 68 with a raised lip 69 located at the proximal end of opening 66. Shield 62 also has a flat distal end 73 that is perpendicular to a longitudinal axis of shield 62.

As shown in Fig. 6, shield 62 is removably-latched in an extended position wherein raised lip 69 on flexible tab 68 of shield 62 is latched to raised latch 71 on flexible arm 70 of shank 63. Shield 62 can be unlatched and slid by the user to another position by applying sufficient force on shield 62 in the direction of butt 74 so that raised latch 71 disengages from raised lip 69. When disengaged, shield 62 is permitted to slide from the extended position, shown in Fig. 6, to a retracted position, shown in Fig. 8.

The cross-sectional view in Fig. 7 of scalpel 60 shown in Fig. 6, shows raised lip 69 of shield 62 latched to raised latch 71 on shank 63. In addition, Fig. 7 shows locking abutment 77 on shank 63, to be described below. When shield 62 is in the extended position, blade 80 is surrounded by shield 62 so that scalpel 60 can be stored or passed between users. Exemplary blade 80, shown in Fig. 6, is substantially similar to the arrow-shaped blade 50 in Fig. 5 and is attached to a proximal end of shank 63 on handle 61.

Fig. 8 is an isometric view of scalpel 60 with slidable shield 62 in the retracted position, wherein blade 80 extends a predetermined distance beyond proximal end 73 of shield 62 and provides a mechanism by which the depth and width of a starter incision is controlled.

In the retracted position, shield 62 is releasably secured in a predetermined position by the pair of shoulders 65 on resilient button 64 mating with one of the pairs of indents 67 on the sides of opening 66. In Fig. 8, shoulders 65 are located in the centrally located pair of indents 67 in opening 66. Fig. 9 is a cross-sectional view of scalpel 60 shown in Fig. 8 and shows the interaction of shoulders 65 on button 64 with the centrally located pair of indents 67 in opening 66.

Shoulders 65, however, could alternatively mate with the pair of indents 67 at the distal end of opening 66 or the pair of indents 67 at the proximal end of opening 66. Each pair of indents 67 correspond to a particular predetermined width and depth incision to be made in a cavity wall for the insertion of a particular predetermined trocar. If the pair of indents 67 located at the distal end of opening 66 mate with'shoulders 65, a wider and deeper incision will be made since more of blade 80 will extend beyond distal end 73 of shield 62. Likewise, if the pair of indents 67 located at the proximal end of opening 66 mate with shoulders 65 a shallower and smaller width incision will be made. Shoulders 65 are moved from one pair of indents 67 to another pair of indents 67 by the user depressing button 64 and sliding shield 62 in the desired direction until shoulders 65 mate with the desired pair of indents 67 in opening 66. Each pair of indents 67, for example, correspond to a predetermined diameter trocar, e.g., 5mm, 10mm, or 12mm, and when the user desires to make a starter incision for one of these trocars the indent corresponding to the specific diameter trocar is mated with shoulders 65 on button 64. In addition, each indent is labeled with an appropriate label (not shown) to permit a user to associate the indent with a particular trocar diameter.

When shield 62 on scalpel 60 is in the retracted position desired by the user, a starter incision is made in a cavity wall by applying pressure in the C direction and forcing blade 80 through the wall. As described above, with respect to arrow-shaped blade 50 in Fig. 5, the starter incision is made by a pair of cutting edges on blade 80. The depth of the incision is controlled by the amount of blade 80 extending beyond proximal end 73 of shield 62, with proximal end 73 limiting the movement of blade 80 in the C direction by proximal end 73 coming into contact with the cavity wall. Therefore, when proximal end 73 contacts the cavity wall, movement in the C direction is prohibited and the starter incision having the desired width and depth, predetermined by the position of shoulders 65 in indents 67 in opening 66, is complete.

When surgical scalpel 60 is in the retracted position shown in Fig. 8, it is capable of efficiently making a controlled width and depth incision so that the proper incision is made for starting a trocar of a predetermined size through the cavity wall. After the desired starter incision has been made in the cavity wall, button 64 is depressed to disengage shoulders 65 from indents 67 and release shield 62 for slidable movement along shank 63 on handle 61. The user then applies pressure to shield 62 toward distal end 73 to slide shield 62 along shank 63 to either the extended position shown in Figs. 6 and 7 or to a fully-extended and locked position shown in Figs. 10 and 11, described below.

Fig. 10 shows scalpel 60 in the fully-extended and locked position, wherein handle 61 and shield 62 are permanently locked together by a locking mechanism. The locking mechanism includes a locking abutment 77 on flexible arm 70 spaced from raised latch 71 that mates with flexible tab 68 having raised lip 69 on shield 62. The use of the locking mechanism is more clearly shown in Fig. 11, wherein raised lip 69 on flexible tab 68 is locked to locking abutment 77 on shank 63. In addition, Fig. 11 shows flexible arm 70 extending down and underneath flexible tab 68 and pressure surface 72 making contact with internal surface 78 of shield 62 to provide stability to shield 62 so that it does not move on shank 63.

When scalpel 60 is in the fully-extended and locked position in Figs. 10 and 11, blade 80 is permanently surrounded by and locked in shield 62 so that shield 62 cannot be retracted towards butt 74 on handle 61, which allows the user to dispose of scalpel 60.

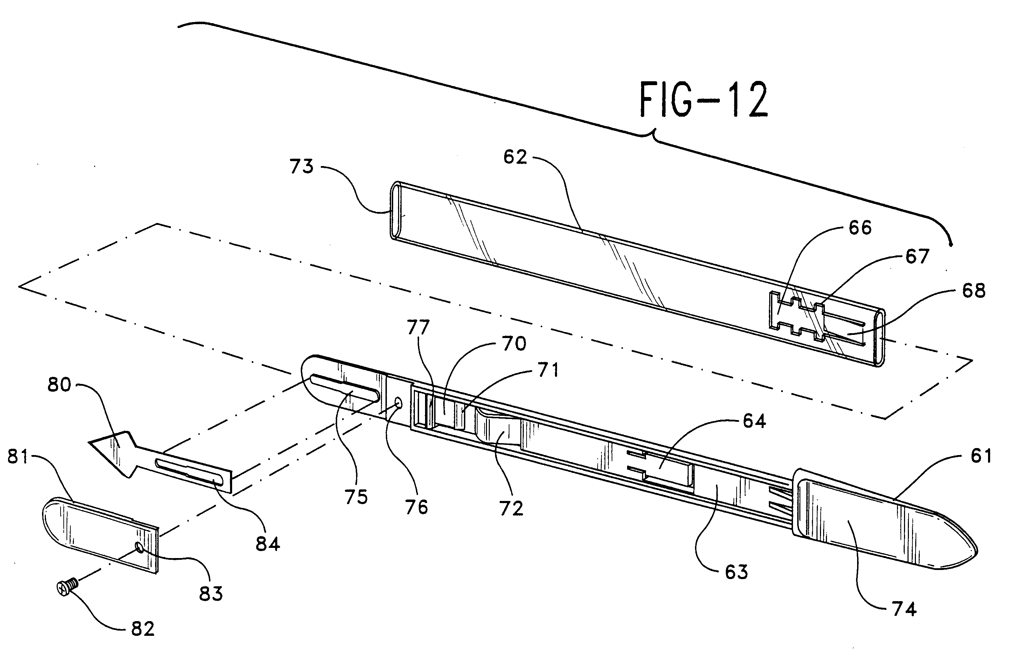

Fig. 12 is an exploded isometric view of scalpel 60 shown in Figs. 6-11, and shows all of the features of scalpel 60 in more detail. For example, Fig. 12 shows an opening 84 in blade 80 being attached to a boss 75 at the distal end of handle 61. A locking plate 81 covers the base of blade 80 to hold blade 80 firmly on shank 63 of handle 61 and is held in position by a pin 82 which passes through a plate hole 83 into a locking hole 76 on shank 63 of handle 61.

Scalpel 60 can be made of surgical steel or plastic material. However, it would be preferable to make the entire scalpel 61, except for blade 80, in plastic for use as a disposable scalpel.

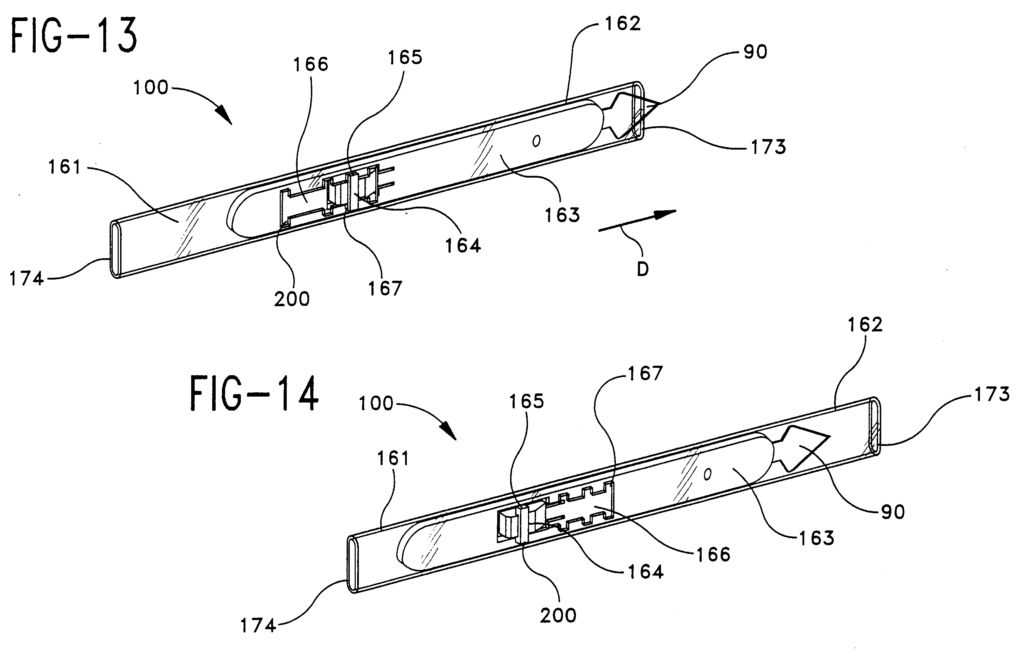

Figs. 13 and 14 show an alternative preferred embodiment of a surgical scalpel 100 according to the present invention. Surgical scalpel 100 includes a blade 90 attached to a shank 163 and a handle portion 161 that serves as a shield 162 to cover blade 90 when not in use: Shank 163 also includes a resilient button 164 having a pair of shoulders 165 extending therefrom and shield 162 includes a longitudinal opening 166 having three pairs of indents 167, each corresponding to a particular predetermined depth and width incision, and a pair of securing indents 200 at the distal end of opening 166.

In Fig. 13 scalpel 100 is shown in an extended position with blade 90 extending beyond distal end 173 of shield 162, and the pair of shoulders 165 mating with one of the pairs of indents 167 in opening 166 on shield 162. Shoulders 165 in combination with indents 167 removably secure shank 163 in proximal relationship with shield 162, so to define the amount of blade 90 that extends beyond distal end 173 of shield 162.

When the user wishes to set the desired width and depth incision using scalpel 100, button 164 is pressed to disengage shoulders 165 from the pair of indents 167 in opening 166 and shank 163 is slide within shield 162 until shoulders 165 mate with another pair of indents 167 in opening 166 to thereby set the scalpel for a new depth and width incision setting. As with the scalpel shown in Figs. 6-12, if a deeper and wider incision is desired the pair of indents 167 at the distal end of opening 166 are used, but if a shallower and narrower incision is desired the pair of indents 167 near the proximal end of opening 166 are used. For example, each pair of indents 167 corresponds to a specific diameter trocar, e.g., 5mm, 10mm, or 12mm, to be inserted into the cavity wall. In addition, each indent is labeled with an appropriate label (not shown) to permit a user to associate the indent with a particular trocar diameter. When shoulders 165 mate with indents 167, blade 90 is fixed in relationship with distal end 173 of shield 162 and thereby provides a mechanism for controlling the depth and width of an incision being made by blade 90.

Fig. 14 shows scalpel 100 in a retracted position with shield 162 extending beyond and covering blade 90. When the user is finished making the starter incision and wishes to secure the scalpel for storage, passing to other personnel, or disposal, button 164 is pressed to disengage shoulders 165 from the pair of indents 167 and shank 163 is moved in the proximal direction of shield 162 until shoulders 165 mate with the pair of securing indents 200 located at the distal end of opening 166.

To move the scalpel from the retracted position in Fig. 14 to the extended position in Fig. 13, the user presses button 164 to disengage shoulders 165 from indents 200 and then slides button 164 towards distal end 173. The desired depth and width of the incision is then set by the user again engaging shoulders 165 on button 164 with one of the three pairs of indents 167 in opening 166, as described above.

Scalpel 100 is used in the extended position shown Fig. 13 by the user holding it with the point of blade 90 in contact with a patient's cavity wall. Pressure is then applied in the D direction, so that blade 90 pierces the cavity wall and twin cutting edges on blade 90 make a controlled incision in the cavity wall. Pressure is applied in the D direction until proximal end 173 of shield 162 makes contact with the cavity wall, at which point the starter incision has the desired depth and width as predetermined by the pair of indents 167 engaged with shoulders 165. After the starter incision has been made the scalpel 100 is extracted from the incision and the desired trocar is inserted through the incision in the cavity wall.

Accordingly, it will be appreciated that the present invention provides a much needed tool for making a controlled width and depth starter incision for inserting a trocar during endoscopic surgery.

In the forgoing discussion, it is to be understood, of course, that the above-described embodiments are simply illustrative of an apparatus embodying the principles and concepts of the present invention. Other suitable variations and modifications could be made to the apparatus described and still remain within the scope of the present invention.

## Claims

1. A surgical scalpel comprising:
a handle having a distal and proximal end and a boss located at the distal end; and
a blade having a head portion with a point, a first and second cutting edge converging to said point, and an opening for receiving said boss when said blade is attached to said handle.

2. A surgical scalpel according to Claim 1, wherein said head portion has a length of at least 14mm.

3. A surgical scalpel according to Claim 1, wherein said head portion has a width of at least 7mm.

4. A surgical scalpel according to Claim 1, wherein said blade includes a foot at the proximal end, and said handle includes a slanted abutment spaced from said boss for receiving said foot of said blade.

5. A surgical scalpel according to Claim 1, wherein said blade has an arrow-shaped point.

6. A surgical scalpel comprising:
a handle;
a shield having a distal end slidably mounted on said handle to slide between an extended position and a retracted position;
means for latching said shield on said handle in the retracted position so that said blade extends beyond said distal end of said shield by a predetermined distance; and
a blade having a first and second cutting edge attached to said handle.

7. A surgical scalpel according to Claim 6, wherein said blade has an arrow-shaped head.

8. A surgical scalpel according to Claim 6, wherein said latching means comprises:
an opening on said shield having a pair of indents; and
a resilient button on said handle having a pair of shoulders,
wherein said shoulders mate with said pair of indents to lock said shield in the retracted position.

9. A surgical scalpel according to Claim 6, further comprising means for locking said shield on said handle in a fully-extended and locked position so that said blade is surrounded by said shield for disposal.

10. A surgical scalpel according to Claim 9, wherein said locking means includes a locking abutment on said handle that engages a raised lip on said shield when said shield is in the fully-extended position.
